# EUROPEAN PATENT SPECIFICATION

(11) **EP 2 592 092 B1**
(45) Date of publication and mention of the grant of the patent: **25.10.2017**
(21) Application number: 12194425.0
(22) Date of filing: 08.02.2008
(51) Int. Cl.: C07K 14/575

(54) **Peptidic and non peptidic ligands for immunodetection of the receptor for urotensin II**
Peptid- und Nichtpeptidliganden zur Immundetektion des Rezeptors für Urotensin II
Ligands peptidiques et non peptidiques pour l'immunodétection du récepteur pour l'urotensine II

(30) Priority: 09.02.2007 IT FI20070032
(43) Date of publication of application: 15.05.2013
(62) Divisional of application: 08708817.5
(73) Proprietor: Novellino, Ettore, 83040 Montemarano (IT); Grieco, Paolo, 80121 Napoli (IT); Caraglia, Michele, 83031 Ariano Irpino (IT); Budillon, Alfredo, 80121 Napoli (IT); Franco, Renato, 80055 Portici (IT)
(72) Inventor: Novellino, Ettore, 83040 Montemarano (IT); Grieco, Paolo, 80121 Napoli (IT); Caraglia, Michele, 83031 Ariano Irpino (IT); Budillon, Alfredo, 80121 Napoli (IT); Franco, Renato, 80055 Portici (IT); Addeo, Santolo Rosario, 80036 Palma Campania (IT)
(74) Representative: Di Donato, Antonio

(56) References cited:
- WO-A-01/37780
- WO-A-01/37856
- GRIECO PAOLO ET AL: "Urotensin-II receptor ligands. From agonist to antagonist activity", JOURNAL OF MEDICINAL CHEMISTRY, vol. 48, no. 23, November 2005 (2005-11), pages 7290-7297, XP002491480, ISSN: 0022-2623
- GRIECO ET AL: "A New, Potent Urotensin II Receptor Peptide Agonist Containing a Pen Residue at the Disulfide Bridge", JOURNAL OF MEDICINAL CHEMISTRY, USAMERICAN CHEMICAL SOCIETY. WASHINGTON, vol. 45, no. 20, 26 September 2002 (2002-09-26), pages 4391-4394, XP002320555, ISSN: 0022-2623
- XIA C ET AL: "Identification of a prostate-specific G-protein coupled receptor in prostate cancer", ONCOGENE, NATURE PUBLISHING GROUP, GB, vol. 20, no. 41, 13 September 2001 (2001-09-13), pages 5903-5907, XP002599902, ISSN: 0950-9232
- PAOLO GRIECO ET AL: "Urotensin II receptor predicts the clinical outcome of prostate cancer patients and is involved in the regulation of motility of prostate adenocarcinoma cells", JOURNAL OF CELLULAR BIOCHEMISTRY, vol. 112, no. 1, 1 January 2011 (2011-01-01), pages 341-353, XP55058332, ISSN: 0730-2312, DOI: 10.1002/jcb.22933

## Description

### Field of the invention

The present invention is referred to the diagnosis and prognosis of prostate adenocarcinoma.

### State of the art

Prostate adenocarcinoma is the fourth cause of death for tumours in Western Countries and represents one of the so-called "big killers ". Advanced and/or metastatic prostate cancer, in its hormone-refractory phase, is poorly responsive to conventional or alternative therapeutic approaches and the early onset of chemotherapy has demonstrated to give an improvement of the survival of this kind of patients. On the basis of these considerations, the definition of the clinical outcome of prostate cancer could be an important issue in the optimization of the therapeutic approaches. The definition of the clinical behaviour and the biological aggressiveness of this tumour is almost completely given by the study of the differentiation of the tumour based upon Gleason score. The latter is determined by the subjective examination of the tumour by pathologist who gives a morphologic score. High Gleason score is not always correlated to a worse prognosis and the finding of new and objective markers of differentiation in prostate cancer is mandatory for the definition of the clinical outcome. Another important issue in the characterization of metastatic prostate cancer is the determination of bone metastases that are not always clearly detectable with the conventional imaging procedures. The development of scintigraphy or PET-based procedures using radio-labelled UTR ligands could be useful in this specific setting in order to discriminate prostate cancer bone metastases.

### Brief description of the figures

Fig. 1 reports the evaluation of UTR on a panel of epithelial and haematological cancer cells.
Fig. 2 shows the immunohistochemical UTR evaluation in prostate adenocarcinoma
Fig. 3 shows the role of UTR in predicting the clinical outcome (survival) of prostate cancer patients independently from Gleason score.
Fig. 4 shows how the UTR expression allows the detection of patients with bad prognosis in Gleason > 7 tumours
Fig. 5 shows how UTR expression predicts the clinical outcome of patients affected by T3- T4 tumours.

### SUMMARY OF THE INVENTION

The application refers to a prognostic method for the human prostatic adenocarcinoma characterized in that the UTR expression is measured on an ex-vivo surgical prostate adenocarcinoma sample from a patient subject to tumor biopsy and/or radical prostatectomy.

### Detailed description of the invention

It was now found that UTR protein is expressed at high levels in human androgen-dependent prostate adenocarcinoma LnCaP cell line after evaluation of UTR expression in a wide panel of human tumour cell lines.

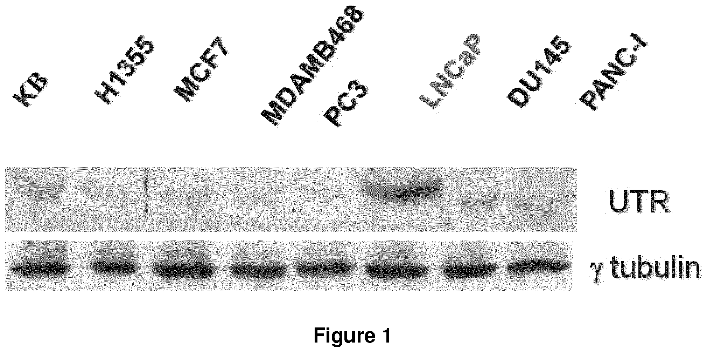

In details, UTR was evaluated in the three "classical" cell lines of human prostate carcinoma, PC3, LNCaP and DU145, by Western Blotting method. UTR is highly expressed in LNCaP cell line while its expression is low or undetectable in the other two cells (Figure 1).

On the basis of these results it was also surprisingly found that UTR is expressed at high levels in prostate adenocarcinoma samples collected from patients subjected to tumour biopsy and/or radical prostatectomy.

The expression of UTR can be evaluated with immunohistochemical methods using an antibody already commercially available. In detail, UTR has been found to be specifically expressed in low Gleason grade prostate
adenocarcinoma while its expression was gradually lost in high Gleason grade and completely undetectable in Gleason 5. The overall survival of the patients was moreover correlated to the expression of UTR that was also able to discriminate the patients with good prognosis from those with bad prognosis in the group of patients with Gleason Grade > 7. UTR is in our series an independent prognostic marker and is strongly correlated to Gleason score and tumour stage. Therefore, UTR appears an objective marker that allows the optimization of surgical treatment and predicts the responsiveness to medical treatments of human prostate adenocarcinoma.

### Example 1

### Immunohistochemical detection of UTR in surgical samples

Tissue samples are fixed in 10% buffered neutral Formalin, included in paraffin and 5µ sections are obtained. All sections then were de-paraffinized in xylene (BioClear, three times for 15 min), rehydrated through a graded alcohol series (95% *alcohol* to discard xylene, followed by 90% and 70% *alcohol* for 30 min) and finally washed in PBS. This buffer was used for all subsequent washes and for dilution of the antibodies. The *slides* were incubated in Cytrate buffer (ph 6.0) in microwave *oven* three times for 5 min in order to unmask antigenic sites. Thereafter, the slides are incubated in 100 µl H₂O₂/ methanol 0,5% v/v for 10 min at room temperature. After incubation the slides are washed twice with TBS/tween 20 buffer for 5 min. In this way the endogenous peroxidase is blocked. Then the slides are incubated in primary antibody (anti-UTR antibody purchased from Santa Cruz, Santa Cruz, Ca) at 1:3000 dilution for 1 h at room temperature. Then the slides are washed twice with TBS/tween 20 buffer for 5 min. The slides are incubated with a secondary goat anti-rabbit antibody linked through a dextrane polymer to horse radish peroxidase. After incubation for 30 min at room temperature the slides are washed twice with TBS/tween 20 buffer for 5 min. The sections are covered with 100 µl chromogen substrate (DAB, 3,3'- Diaminobenzidin and 2,5-3% H₂O₂) and incubated for 10 min at room temperature at dark. Thereafter, the slides are washed again in distilled water to discard Diaminobenzidin and counterstained with ematoxilin for about 1 min followed by washing in water for at least 5 min. The slide were dehydrated through a graded alcohol series, clarified in xylene and mounted for visualization at optical microscope (Zeiss).

The UTR expression has been evaluated on 200 in vivo samples collected from patients affected by prostate adenocarcinoma. UTR expression has been correlated with different clinical and pathological parameters including Gleason score, androgen receptor expression, age and TNM.

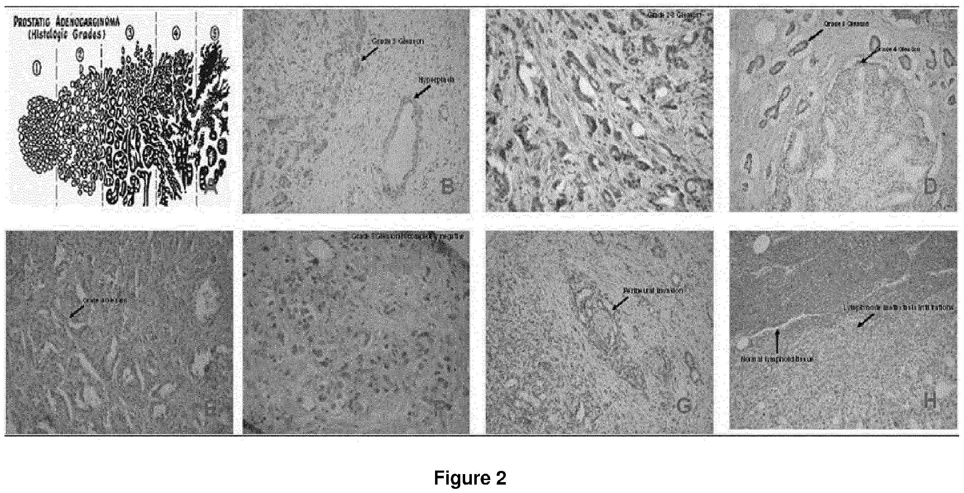

The expression was very high in Gleason 2-3 (C and D) and absent in Gleason 5 (F). UTR was expressed in prostate tumour cells infiltrating a nerve (G) and a node (H) (Figure 2).

The correlation between UTR expression and low Gleason score was statistically significant (p=0.001) while it did not correlate with either age (p=0.4) or stage (p=0.007) (Table 1).

**TABLE 1**

| **UTR expression in relation to clinical and pathological parameters in a series of 195 prostate cancer patients** | | | | |
|---|---|---|---|---|
| | **Total** | **Low** | **High** | ***p* value** |
| | | **n (%)** | **n (%)** | |
| | **195** | **106 (54)** | **89 (46)** | |
| **Age (yr)** | | | | |
| ≤70 | 109 | 62 (57) | 47 (43) | |
| >70 | 86 | 44 (51) | 42 (49) | 0.4 |
| | | | | |
| **Tumor stage*** | | | | |
| pT2 | 67 | 29 (43) | 38 (57) | |
| pT3 | 62 | 36 (58) | 26 (42) | |
| pT4 | 66 | 41 (62) | 25 (38) | 0.07 |
| **Gleason score*** | | | | |
| Low | 50 | 16 (32) | 34 (68) | |
| Medium | 70 | 34 (49) | 36 (52) | |
| High | 75 | 56 (75) | 19 (25) | 0.001 |

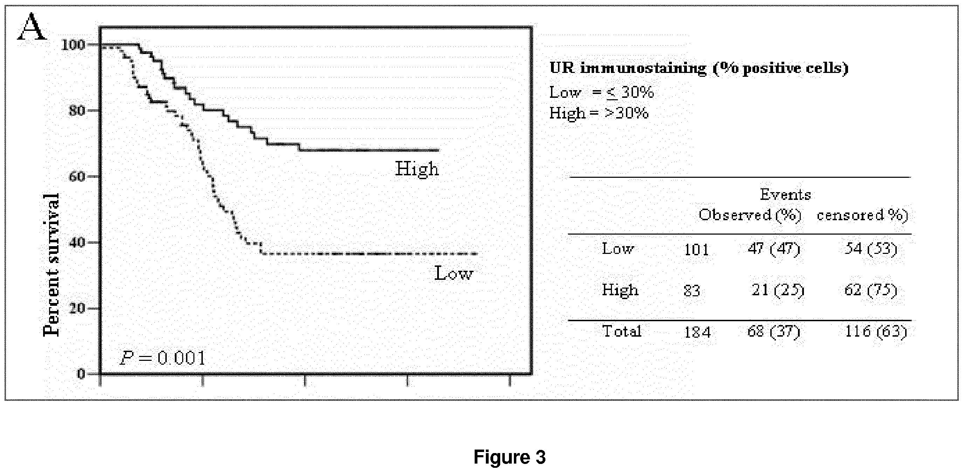

The overall survival of patients having more than 30% of cells expressing UTR was significantly higher than those with low UTR expression (p= 0.001) (Figure 3) that was also an independent prognostic factor together with stage Table 2.

**TABLE 2**

| **Contribution of various potential prognostic factors to overall survival by Cox regression analysis in prostate cancer patients** | | | |
|---|---|---|---|
| Variable | *Risk* ratio | *95% confidence* interval | *p* value |
| Age§ | 1.412 | 0.876-2.276 | 0.157 |
| Gleason score* | 1.012 | 0.547-1.907 | 0.948 |
| Tumor stage# | 3.296 | 1.712.-6.347 | 0.001 |
| UR** | 2.250 | 1.323-3.828 | 0.003 |
| § The risk ratio is given as older versus younger patients (cut-off = 70 years) | | | |
| *The risk ratio is given as higher (>7) versus lower Gleason score. | | | |
| #The risk ratio is given as higher (pT4) versus lower stage cancers. | | | |
| **The risk ratio is given as low (≤ 30% positive cells) versus high (>30%) expressor tumors. | | | |

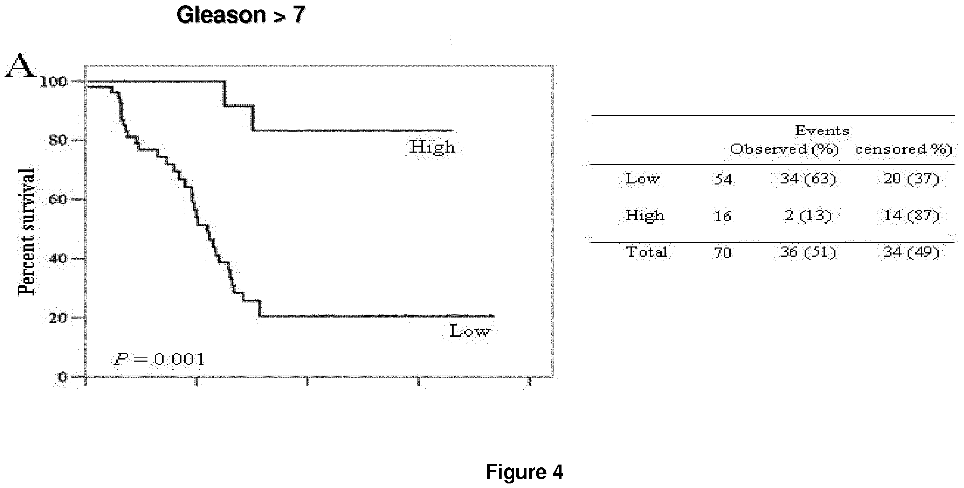

Interestingly, UTR was able to discriminate the sub-group of patients with good prognosis when Gleason score was more than 7 (p=0.001) (Figure 4).

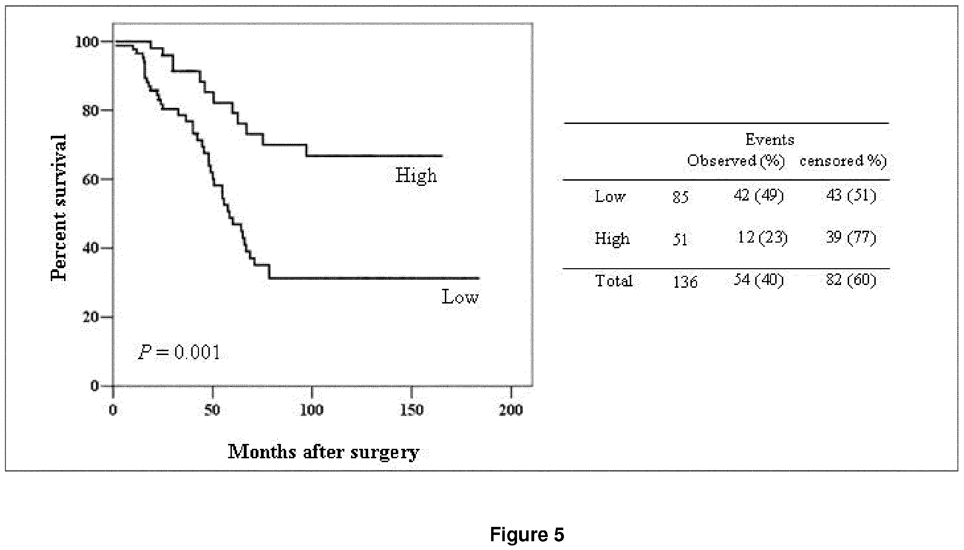

UTR preserved its discriminating function also in pT2-pT3 and pT3-pT4 patient subgroups (Figure 5). On the other hand, the correlation between UTR and androgen receptor expression was not significant (p>0.05).

The measurement of UTR expression in prostate cancer is useful to establish the prognostic score of the patients per se and independently (being UTR and independent prognostic factor) from Gleason score that remains a subjective morphologic parameter of evaluation that can change from a pathologist to another. In fact, this kit is based on semi-quantitative parameters (more or less than 30% of cells positive for UTR expression) and, therefore, provides subjective criteria of evaluation of the grade of differentiation of the tumor.

The evaluation of UTR expression is also useful to discriminate the patients with good prognosis in the subset group with Gleason score more than 7. The definition of the prognosis of this subset of patients is an important and debated issue. In fact, part of these patients continues to have a long-lasting androgen-responsive disease while another group has a disease rapidly developing in aggressive and hormone-refractory cancer. Therefore, the early definition of the clinical outcome of these patients has important consequences on the choice of the optimal self-tailored therapeutic strategy. The availability of a cheap and easy maker assay that can drive the choice of the optimal therapy will have a rapid and wide diffusion in the clinical practice.

## Claims

1. Prognostic method for the human prostatic adenocarcinoma **characterized by** measuring the expression of Urotensin-II receptor (UTR) on an ex-vivo surgical prostate adenocarcinoma sample from a patient subject to tumor biopsy and/or radical prostatectomy.

2. Prognostic method according to claim 1 wherein said expression is determined independently from Gleason score.

3. Prognostic method according to claim 1, wherein adenocarcinoma is characterized for patients having a Gleason grade higher than 7.

## Patentansprüche

1. Prognostische Verfahren für das menschliche Adenokarzinom der Prostata **gekennzeichnet durch** die espression von Urotensin 2 Rezeptor auf einer ex-vivo chirurgische Prostata Adenokarzinom Probe von einem Patienten zu Tumorbiopsie und/oder radikale Prostatektomie.

2. Prognostische Verfahren nach Anspruch 1, wobei der Ausdruck aus dem Gleason Score bestimmt.

3. Prognostische Verfahren nach Anspruch 1, wobei das Adenokarzinom für Patienten mit einem Gleason-Grade höher als 7 characterisiert ist.

## Revendications

1. Méthode pronostique pour évaluer le développement de l'adénocarcinome prostatique par la mesure de l'expression du récepteur (UTR) de l'Urotensine II sur un échantillon de cellules « ex-vivo » d'adénocarcinome de la prostate, prélevées de la biopsie et/ou de la prostatectomie de la tumeur du patient.

2. Méthode pronostique selon la revendication 1 où dite expression est **indépendaamment** déterminée par le Gleason Score

3. Méthode pronostique selon la revendication 1 où l'adénocarcinome est caractérisé pour les patients qui ont un Gleason Score plus élevé de 7.
